# EUROPÄISCHE PATENTANMELDUNG

(11) **EP 3 701 916 A1**
(43) Veröffentlichungstag der Anmeldung: **02.09.2020**
(21) Anmeldenummer: 20000087.5
(22) Anmeldetag: 27.02.2020
(51) Int. Cl.: A61F 7/00, A61F 7/02, A61N 2/00, A61H 23/00

(54) **MULTIFUNKTIONALE, TEMPERATURGESTEUERTE KÖRPERAUFLAGE (KOMPRESSE) ZUR KÄLTE- UND WÄRMEBEHANDLUNG VON GEWEBEFLÄCHEN**

(30) Priorität: 01.03.2019 DE 202019001024 U
(71) Anmelder: Kett, Reinhold, 85258 Weichs (DE)
(72) Erfinder: Kett, Reinhold, 85258 Weichs (DE)

(57) **Zusammenfassung**

Die multifunktionale Körperauflage dienst zur automatisierten Erzeugung einer Kühl- oder Wärmewirkung an Körper- bzw. Gewebeoberflächen, wobei die Körperauflage in form einer Kompresse aus einer dünnen, hochflexiblen und wärmeleitfähigen Kontaktfolie besteht, welche von einem Trägermedium (flüssig oder gasförmig) durchströmt wird. Die einstellbare Temperatur des Trägermediums wird von einem Steuergerät erzeugt und kann beliebig in definierbaren Zeiträumen konstant gehalten oder dynamisch während der Behandlungszeit verändert werden.

Der Sinn der Erfindung besteht darin, herkömmliche und umständliche Behandlungsmethoden mittels Kühl- oder Wärmepackungen zu ersetzen und so für Arthrose- oder Rheuma-Erkrankungen, zur Schmerzlinderung bei postoperativen Wundbehandlungen, Schwellungen, Entzündungsreaktionen, etc. eine komfortable und automatisierte Anwendung ohne Unterbrechung des Behandlungszeitraumes zu ermöglichen.

Gleichzeitig kann die Körperauflage mit zusätzlichen Behandlungsmethoden (Licht-, Magnetfeld-, Vibrationstherapie) kombiniert werden, indem modulartige, flächengleiche Elemente in Sandwichbauweise mit der Kompresse verbunden werden können, welche ebenfalls über das Steuergerät entsprechend der Anwendung betrieben werden. Beim Trägermedium "Luft" kann an das Steuergerät anstatt der Körperauflage alternativ auch eine Beatmungsmaske angeschlossen werden.

## Beschreibung

Die Erfindung betrifft eine Körperauflage, welche als Kühlkompresse, als Wärmekompresse oder in Kombination als Wechseltemperaturkompresse an unterschiedlichen Körperregionen einsetzbar ist. Gleichzeitig besteht auch bei Bedarf die Möglichkeit einer kombinierbaren Magnetfeldtherapie oder einer Infraschall-Stoßwellentherapie, einer Vibrationsmassage oder einer Lichttherapie des Gewebes im Umfeld der Körperauflage.
Prinzipiell besteht die Kompresse aus vorzugsweise flexiblem Kunststoffmaterial, welche so gestaltet ist, dass sie von einem flüssigen oder gasförmigen Trägermedium durch zweckmäßig mäanderförmig angeordnete Kanäle durchströmt werden kann.
Die Temperatur der Flüssigkeit wird elektronisch gesteuert oder geregelt und kann beispielsweise durch den Einsatz von Peltier-Elementen einen Temperaturbereich von -10 Grad Celsisus bis + 50 Grad Celsius erreichen.
Um einen schnellen Temperaturwechsel der Körperauflage von KALT nach WARM und umgekehrt zu erreichen, ist in die Auflagefläche zusätzlich ein Heizelement beispielsweise ein Heizdraht eingebaut, welcher ebenfalls temperaturgesteuert oder geregelt wird.
Zur Temperaturmessung und Temperaturregelung der Körperauflage ist in die Kompresse ein entsprechender Sensor integriert, welcher mit dem Steuergerät verbunden ist.
Die Kompresse ist mit einem thermisch isolierten Schlauchsystem zum Transport des Trägermediums (flüssig oder gasförmig) mit dem Steuergerät verbunden, welches im Bedarfsfall mittels totraumfreien Ventil-Schlauchkupplungen getrennt, bzw. umgesteckt werden kann, was bei flüssigem Trägermedium einen Flüssigkeitsverlust verhindert und über ein Verteilsystem den gleichzeitigen Anschluss mehrerer unterschiedlicher Körperauflagen ermöglicht.
In Kombination zu einer Temperaturbehandlung der Körperregionen ist gleichzeitig auch eine Magnetfeldtherapie, eine Infraschall-Stoßwellentherapie eine Vibrationsmassage oder eine Lichttherapie im Umfeld der Kompresse möglich. Hierzu wird flächengleich zur Kompresse im Sandwichverfahren an Stelle der oberseitigen Thermo-Isolierung alternativ ein zusätzliches Magnetspulenfeld oder ein Infraschallgeber oder ein Massage-Panel oder ein Licht-Panel auf die Unterseite der Kompresse angebracht und ebenfalls elektrisch mit dem Steuergerät verbunden.
Die temperaturgesteuerte Körperauflage ermöglicht zahlreiche Anwendungsfälle zur Behandlung in den Bereichen der Blutgefäßerkrankung, akute und chronische Schmerzlinderung, Reduzierung akuter Schwellungen, Fiebersenkung, Entzündungshemmung, Heilbeschleunigung, etc.

Bisherige Kälte- oder Wärmebehandlungen von Körperregionen werden mittels Cool-Packs, Kühlgels, Eispackungen, Wärmepackungen, etc. durchgeführt. Diese erfordern immer das Vorhalten entsprechender Kühl- bzw. Wärmemittel und Speichermaterialien, welche auch im Laufe der Behandlung alsbald durch den Temperaturverlust ausgetauscht, bzw. erneuert werden müssen. Des Weiteren kann mit den herkömmlichen Methoden die Oberflächentemperatur der Kompresse weder eingestellt noch über den gesamten Behandlungszeitraum konstant gehalten werden

Der Einsatz der beschriebenen temperaturgeregelten Körperauflage ermöglicht eine wählbare Oberflächentemperatur, sowohl im kalten als auch warmen Bereich, welche über den gesamten, einstellbaren Behandlungszeitraum konstant gehalten werden kann und auch keine Unterbrechung der Behandlung durch einen Wechsel der Kompressen erfordert. Die zusätzlichen Anwendungen mit Magnetfeld, Infraschall, Vibration oder Licht ermöglichen zeitgleich und in einem Gerät verschieden kombinierbare Therapien. Als weiteren Vorteil ist das beschriebene und sehr komfortable System sowohl stationär als auch mobil einsetzbar und kann in Kliniken, von Physiotherapeuten, von Heilpraktikern und Notärzten sowie in Altenheimen und bei Sportveranstaltungen, etc. verwendet werden.

Im Falle, dass als Trägermedium lediglich Luft verwendet wird, könnte das Konzept als weitere Anwendungsmöglichkeit in Form eines Beatmungsgerätes z.B. für "Apnoe-Patienten" eingesetzt werden. Hierzu ist statt der Kompresse eine Maske (Vollgesichtsmaske oder Nasenmaske) an das Steuergerät anzuschließen, welche die vom Steuergerät erwärmte oder abgekühlte Luft mit entsprechendem Druck zur Atembehandlung bereitstellt. Neben Temperatur und Druckeinstellung kann auch eine Atemluftbefeuchtung oder eine zusätzliche Atemluftanreicherung mit reinem Sauerstoff mittels extern anschließbarer Komponenten erfolgen.

Im Folgenden wird die Erfindung näher beschrieben:

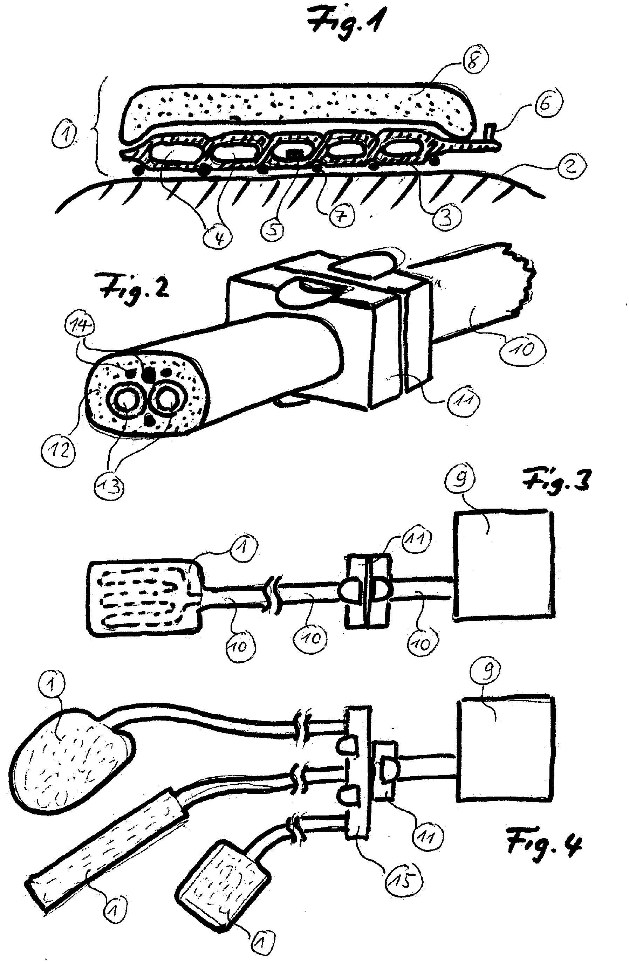

Fig.1 zeigt einen Querschnitt der Körperauflage (1) mit den Hohlräumen (4) für das Tägermedium (llüssig oder gasförmig), welche zur optimalen Anpassung an verschiedene Körperoberflächen (2) mittels eines flexiblen Kunststoffmaterials realisiert ist, wobei die Körperauflagefläche (3) der Kompresse (1) aus einer nur wenige Mikrometer dicken und wärmeleitenden Spezialfolie besteht, um den Temperatur-Übergangswiderstand zur Körperoberfläche (2) zu verringern. Auf der Oberseite der Körperauflage (1) ist eine Isolationsschicht (8) zur Vermeidung eines Temperaturverlustes abnehmbar angebracht, dergestalt, dass an der gleichen Stelle alternativ zusätzliche andere Auflage-Panles, z.B. Magnetfeld-, Infraschall- Vibrations-Panels mit der erforderlichen elektrischen Kontaktierung (6) angebracht werden können. Im Inneren der Hohlräume (4) ist mindestens ein Temperatursensor (5) installiert, welcher über das Schlauchsystem (10) mit dem Steuergerät (9) verbunden ist. Mittels Peltier-Elementen kann das Steuergerät (9) eine einstellbare Temperatur des flüssigen oder gasförmigen Trägermediums in einem Bereich von Minus 10 Grad Celsius bis Plus 50 Grad Celsius erzeugen, welche durch ein Pumpensystem im Steuergerät (9) bzgl. Durchflussgeschwindigkeit, Durchflussmenge und Systemdruck geregelt wird. Wie aus Fig. 2 ersichtlich, erfolgt die Verbindung zur Kompresse (1) mittels eines Schlauchsystems (10), welche spezielle Steckkupplungen (11) aufweisen. Bei Verwendung eines flüssigen Trägermediums kommen totraumfreie Kupplungen zur Anwendung, um bei Abtrennung und Wiederanschließen Lufteinschlüsse und Blasenbildung im Hydrauliksystem zu vermeiden. In die thermische Isolierung (12) von Schlauchsystem (10) und der Kupplungen (11) sind alle erforderlichen elektrischen Verbindungsleitungen (14) zwischen Steuergerät (9) und Körperauflage (1) enthalten, wobei mit den Kupplungen (11) die Schlauchleitungen für Vor- und Rücklauf (13) und Elektroleitungen (14) gleichzeitig steckbar sind. Eine prinzipielle Anordnung des Gesamtsystems ist in Fig. 3 gezeigt. Ferner ist die das Kupplungsteil (11) so ausgeführt, dass ein Mehrfachanschluss von Körperauflagen (1) über einen Mehrfachverteiler (15) ermöglicht wird, wie in Fig.4 dargestellt. Ebenso sind die Körperauflagen (1) in Form und Größe an verschiedene Körperregionen angepasst, z.B. Fläche, Gelenke, etc. wie ebenso aus Fig. 4 ersichtlich.
Mit dem multifunktionalen Körperauflage (1) sind verschiedene Therapievarianten möglich:
A. Kühlen der Körperoberfläche mit zirkulierendem Trägermedium (flüssig oder gasförmig)
B. Erwärmen der Körperoberfläche mit zirkulierendem Trägermedium
C. Abwechselndes Kühlen mit Trägermedium durch STOP der Zirkulation und schnelles Erwärmen mit Heizdraht und umgekehrt (Wechselbad-Funktion)
D. Kombinationen von Kühlen bzw. Erwärmen mit zusätzlichen Panels für Magnetfeld, Infraschall, Vibration, Lichtwellen

### Bezugszeichenliste

- 1: Körperauflage, Kompresse
- 2: Körperoberfläche
- 3: Körperauflagefläche
- 4: Hohlräume für das Trägermedium (Flüssigkeit oder Gas)
- 5: Temperatursensor
- 6: Elektrokontakte für zusätzliche Panels
- 7: Heizdrahtschleife
- 8: Thermische Isolation der Kompresse
- 9: Steuergerät
- 10: Schlauchsystem
- 11: Schlauchkupplung (hyrdraulisch bzw, pneumtatisch) mit Elektro-Kontakten
- 12: Thermische Isolation des Schlauchsystems
- 13: Vor- und Rücklaufkanal des Trägermediums
- 14: Elektrokabel
- 15: Mehrfachverteiler

## Patentansprüche

1. Körperauflage zur Erzeugung einer Kühl- und/oder Wärmewirkung an Körperoberflächen (2) **dadurch gekennzeichnet, dass** die Körperauflagefläche (3) aus einer dünnen, hochflexiblen und wärmeleitfähigen Folie besteht, welche zur Erzeugung einer einstellbaren Temperatur an der Körperauflagefläche (3), Hohlräume (4) aufweist, die von einem temperaturgesteuerten oder geregelten Trägermedium (flüssig oder gasförmig) durchströmt werden, wobei die Temperatur der Körperauflagefläche (3) von einem Steuergerät (9) über einen definierbaren Zeitraum konstant haltbar ist.

2. Körperauflage nach Anspruch 1, **dadurch gekennzeichnet, dass** mittels des Steuergerätes (9) der Systemdruck, die Fließgeschwindigkeit und die Temperatur des Trägermediums überwach- und regelbar ist und somit auch entsprechende Sicherheitsfunktionen im Wesentlichen bei System-Druckverlust, Über- oder Untertemperatur des Trägermediums oder Temperaturverlauf an der Körperauflagefläche (3) durchführbar sind.

3. Körperauflage, **dadurch gekennzeichnet, dass** die Körperauflage (1) durch ein thermisch isoliertes Schlauchsystem (10) semipermanent mit dem Steuergerät (9) verbunden ist, welches bei flüssigem Trägermedium mittels totraumfreier Ventil-Hydraulikkupplungen (11) getrennt, verbunden, bzw. auf einen Mehrfachverteiler (15) umsteckbar ist, um mehrere auch unterschiedliche Formen von Kompressen gleichzeitig zu betreiben.

4. Körperauflage, **dadurch gekennzeichnet, dass** die Oberseite der Kompresse (1) mit einer semipermanenten, abnehmbaren Isolationsschicht (8) gegen Temperaturverlust versehen ist.

5. Körperauflage, **dadurch gekennzeichnet, dass** mittels des Steuergerätes (9) nur eine Kühlwirkung oder nur eine Wärmewirkung durch das Trägermedium an der Körperauflagefläche (3) erzielbar ist, oder eine schnelle Wechselwirkung von Kühlwirkung mittels kaltem Trägermedium und Wärmewirkung mittels eines integrierten temperaturgeregelten Heizelements bevorzugt einer Heizdrahtschleife (7).

6. Körperauflage, **dadurch gekennzeichnet, dass** anstelle der thermischen Isolationsschicht (8) mittels der Elektrokontakte (6) optional ein Magnetfeld-, Infraschall-, oder Vibrationsgeber installierbar ist, welcher über die im Schlauchsystem (10) integrierte Elektrokabel (14) mit dem Steuergerät (9) verbunden ist.
